(19) Europäisches Patentamt
European Patent Office
Office européen des brevets

(11) Publication number: 0 103 877 B1

(12) EUROPEAN PATENT SPECIFICATION

(45) Date of publication of patent specification: 07.01.87

(51) Int. Cl.[4]: C 07 H 11/04, C 07 H 15/04, C 07 H 13/04, A 61 K 31/70

(21) Application number: 83109238.2

(22) Date of filing: 17.09.83

(54) **Phosphate derivatives, process for preparation thereof and pharmaceutical compositions of the same.**

(30) Priority: 21.09.82 GB 8226869

(43) Date of publication of application: 28.03.84 Bulletin 84/13

(45) Publication of the grant of the patent: 07.01.87 Bulletin 87/02

(84) Designated Contracting States: BE CH DE FR GB IT LI LU NL SE

(58) References cited: DE-A-2 619 686

(73) Proprietor: **Fujisawa Pharmaceutical Co., Ltd.**
**3, Doshomachi 4-chome Higashi-ku**
**Osaka-shi, Osaka 541 (JP)**

(72) Inventor: **Teraji, Tsutomu**
**No. 20-6, Kofudai 6-chome**
**Toyono-cho Toyono-gun Osaka 563-01 (JP)**
Inventor: **Todo, Eishiro**
**No. 2-2-304, Kitamidorigaoka 1-chome**
**Toyonaka-shi Osaka 560 (JP)**
Inventor: **Shimazaki, Norihiko**
**No. 4-10-303, Kamishinden 4-chome**
**Toyonaka-shi Osaka 565 (JP)**
Inventor: **Oku, Teruo**
**No. 2-23-406, Wakayamadai 2-chome**
**Shimamoto-cho Mishima-gun Osaka 618 (JP)**
Inventor: **Namiki, Takayuki**
**No. 8-22, Wakabaso-1 Sakurai 3-chome**
**Minoo-shi Osaka 562 (JP)**

(74) Representative: **Türk, Dietmar, Dr. rer. nat. et al**
**Türk, Gille + Hrabal Patentanwälte Bruckner Strasse 20**
**D-4000 Düsseldorf 13 (DE)**

EP 0 103 877 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to phosphate derivatives. More particularly, it relates to new phosphate derivatives which have antitumor activity, to a process for preparation thereof, and to pharmaceutical compositions comprising the same for therapeutical treatment of cancer in human being and animals.

Accordingly, one object of this invention is to provide new and useful phosphate derivatives.

Another object of this invention is to provide a process for preparation of the phosphate derivatives.

A further object of this invention is to provide useful pharmaceutical compositions comprising said phosphate derivatives as an antitumor agent.

The phosphate derivatives of the present invention are novel and include the compounds of the formula [I]:

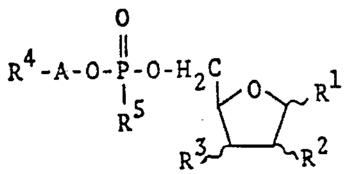

[I]

wherein $R^1$, $R^2$, and $R^3$ are each hydroxy, $C_1$—$C_{25}$ alkoxy, $C_1$-$C_{25}$ alkanoylamino or hydroxy group which is protected by a conventional protective group for hydroxy;

A is $C_2$—$C_6$alkylene;

$R_4$ is tri($C_1$—$C_6$alkyl)-ammonio, 5,6 or 7-membered cyclic ammonio or 5,6 or 7-membered cyclic amino; and

$R^5$ is oxido anion or hydroxy;

and pharmaceutically accepted salts thereof.

In the above and subsequent description of the present specification, suitable examples and illustrations of the various definitions to be included within the scope of the invention are explained in details as follows.

The term "lower" is intended to mean 1 to 6 carbon atom(s).

"Alkoxy" for $R^1$, $R^2$ and $R^3$ is straight or branched one containing 1 to 25 carbon atoms and examples are methoxy, ethoxy, propoxy, isopropoxy, butoxy tert-butoxy, pentyloxy, hexyloxy, heptyloxy, octyloxy, nonyloxy, decyloxy, undecyloxy, dodecyloxy, tridecyloxy, tetradecyloxy, pentadecyloxy, hexadecyloxy, heptadecyloxy, octadecyloxy, nonadecyloxy, eicosyloxy, heneicosyloxy, docosyloxy, tricosyloxy, tetracosyloxy and pentacosyloxy.

"Alkanoyl" moiety in the "alkanoylamino" for $R^1$, $R^2$ and $R^3$ is straight or branched one containing 1 to 25 carbon atoms and examples are formyl, acetyl, propionyl, butyryl, isobutyryl, valeryl, isovaleryl, pivaloyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, octadecanoyl, nonadecanoyl, eicosanoyl, heneicosanoyl, docosanoyl, tricasanoyl, tetracosanoyl and pentacosanoyl.

Examples of a conventional protective group for hydroxy for $R^1$, $R^2$ and $R^3$ are ar(lower)alkyl (e.g. benzyl, p-methoxy-benzyl, benzhydryl, trityl, trimethoxytrityl, lower alkanoyl as mentioned before, substituted lower alkanoyl (e.g. trifluoroacetyl, chloroacetyl), lower alkoxycarbonyl (e.g. methoxycarbonyl, trichloroethoxycarbonyl, tert-butoxycarbonyl), aroyl (e.g. benzoyl, toluoyl, xyloyl, naphthoyl), ar(lower)alkanoyl (e.g. phenylacetyl, phenylpropionyl, phenylbutyryl), aralkoxycarbonyl (e.g. benzyloxycarbonyl), and two adjacent hydroxy groups may be protected as a cyclic acetal (e.g. methyleneacetal, ethylideneacetal, benzylideneacetal, isopropylideneacetal and cyclohexylideneacetal).

"Alkylene" for A is straight or branched one containing 2 to 6 carbon atoms and examples are ethylene, trimethylene, propylene, tetramethylene, pentamethylene and hexamethylene.

Examples for tri($C_1$—$C_6$ alkyl)-ammonio for $R^4$ are triethylammonio, dimethylethylammonio, diethylmethylammonio, tripropylammonio, dimethylpropylammonio, tributylammonio, tripentylammonio and trihexylammonio.

5,6 or 7-membered cyclic ammonio for $R^4$ is a 5,6 or 7-membered heterocyclic ring containing at least one quarternary nitrogen atom such as oxazolio, isoxazolio, thiazolio, isothiazolio, 3H-imidazolio, 2H-imidazolio, 2H-pyrazolio, 2H-1,2,4-triazol-1-io, pyridinio, pyrazinio, pyrimidinio, pyridazinio, 1,2,4-triazin-1-io and 2H-azepinio, among which a more preferable one is a 6-membered heterocyclic ring, and the most preferable one is pyridinio.

5,6 or 7 membered cyclic amino for $R^4$ is a 5,6 or 7-membered heterocyclic ring containing at least one tertiary nitrogen atom such as 1-pyrrolyl, 1-pyrrolinyl, 1-pyrrolidinyl, 1-imidazolyl, 1-pyrazolyl, piperidino, morpholino, 1-piperazinyl, 2-imidazolin-1-yl, 2-pyrazolidinyl, 1-homopiperazinyl, homopiperidino and homomorpholino, among which a more preferable one is a 6 membered heterocyclic ring, and the most preferable one is 1-piperazinyl.

The above cyclic ammonio or cyclic amino group for $R^4$ may be substituted with lower alkyl (e.g. methyl, ethyl, propyl, isopropyl, butyl, isobutyl, sec-butyl, tert-butyl, pentyl, hexyl).

It is to be noted that the compound having a cyclic amino group for $R^4$ and hydroxy group for $R^5$ may be named as an intramolecular salt such as a cyclic ammonio, for example, 1-pyrrolio, 1-piperidinio, 4-morpholinio, 1-piperzinio and 1-imidazolinio.

It is to be understood that there may be one or more stereoisomeric mixture such as optical isomer(s) due to asymmetric carbon atom(s) in the molecules, and these isomers are also included within the scope of the present invention. For instance, the compounds [I] include the ones where each substituent $R^1$, $R^2$ and $R^3$ being bonded below or above the plane of the tetrahydrofuran ring of the compounds [I] such as α and β isomer of xylofuranose, ribofuranose, arabinofuranose, lyxofuranose and the derivatives thereof.

Suitable pharmaceutically acceptable salts of the compounds [I] are conventional non-toxic salts and may include an acid addition salt such as an inorganic acid addition salt (e.g., hydrochloride, hydrobromide, sulfate, phosphate), an organic acid addition salt (e.g., oxalate, maleate, lactate, tartrate, fumarate, methanesulfonate, benzenesulfonate, toluenesulfonate), or a salt with an amino acid (e.g. arginine aspartic acid, glutamic acid and a salt with a base such as alkali metal salt (e.g., sodium salt, potassium salt).

The compounds (I) of the present invention can be prepared by the following process.

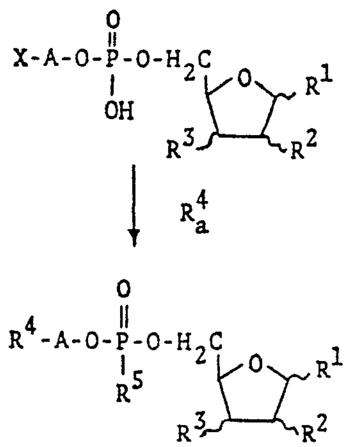


[II]

[III]

[I]

wherein $R^1$, $R^2$, $R^3$, A, $R^4$ and $R^5$ are each as defined above,

X is halogen, and

$R^4_a$ is tri($C_1$—$C_6$ alkyl) amine or 5,6 or 7-membered cyclic amine.

That is, the compounds [I] and salts thereof can be prepared by reacting a compound [II] or its salt with an amine compound [III] or its salt.

In the above formula [II] X is halogen (e.g. fluorine, chlorine, bromine or iodine).

Suitable "alkylamine or cyclic amine" for $R^4_a$ is the one corresponding to tri($C_1$—$C_6$) alkylammonio, cyclic ammonio or cyclic amino for $R^4$ as illustrated before.

A suitable salt of the compound [II] is an alkali metal salt as exemplified before for the compound [I] . A suitable salt of the compound [III] is an acid addition salt as exemplified before for the compound [I].

This reaction is usually carried out in a solvent such as acetone, methanol, tetrahydrofuran, chloroform, benzene or any other solvent which does not adversely affect the reaction. When the compound [III] or its salt is liquid, it can also be used as a solvent.

The reaction temperature is not critical and the reaction is preferably carried out under warming or heating.

The compound [I] may occasionally be obtained as a salt with the hologenide residue ($X^-$ anion).

The above salt can optionally be transformed to another salt or the corresponding intramolecular salt by a conventional method, such as treatment with an ion-exchange resin, or treatment with silver ion.

The starting compounds [II] and their salts are novel and they can be prepared by the methods as shown in the Examples or the methods chemically equivalent thereto.

The following pharmacological test data show that the compounds [I] of the present invention exhibit high anti-tumor activity.

Test Method

Groups of eight female BALB/c mice, aged 8—9 weeks and weighing 18.0—22.5 g were used.

Fibrosarcoma Meth A (hereinafter referred to as Meth A) was successively transferred every 7 days into BALB/c mice by intraperitoneal inoculation of the ascites cells thereof and the Meth A in the ascites cells as harvested 6 or 7 days after the inoculation was used as tumor cells.

Each of the BALB/c mice was inoculated intrapleurally with $5 \times 10^5$ Meth A cells in 0.1 ml Hanks solution.

The test compound was dissolved in phosphate buffer saline solution, and was injected into pleural cavity to each of the mice in doses of 100 µg/0.05 ml/mouse three times, i.e. 14 days before, and 1 hour and 3 days after tumor implantation.

The control group was given the vehicle alone in the same way.

The antitumor activity of the test compound was estimated by comparing mean survival time of the two groups.

T: Mean survival time of the medicated group
C: Mean survival time of the control group

Test Compound
Ex 4-(3)

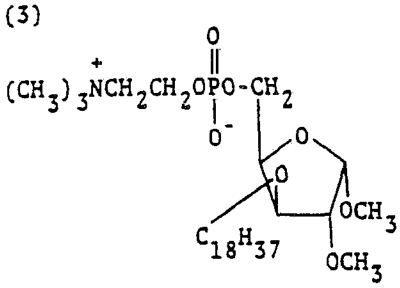

Ex 6-(7)

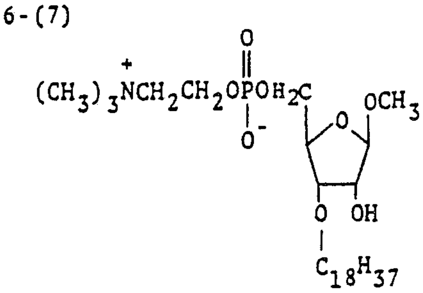

Control Compound

(rac)-1-O-Octadecyl-2-O-methyl-glycerol-3-phosphorylcholine which is decribed in British Patent No. 1583661.

Test Results

| Compounds | Anti-tumor activity *a (%) |
|---|---|
| Ex 4-(3) | 408 |
| Ex 6-(7) | 383 |
| Control Compound | 302.8 |

*a: T/C × 100

It is apparent from the above test results that the compound [I] of the present invention is useful as an antitumor agent.

The effective ingredient may usually be administered at a dose of 0.1 mg/kg to 500 mg/kg, 1 to 4 times a day in a preparation such as tablet, granule, powder, capsule, syrup, injection and suppository. However, the above dosage may be increased or decreased according to the age, weight or condition of a patient or the administration method.

The above mentioned pharmaceutical preparations can be prepared in a conventional manner by using conventional carriers and additives.

The present invention is illustrated in more detail by the following Examples.

Example 1

(1) A mixture of methyl 2-O-methyl-β-D-xylofuranoside (3.45 g), trityl chloride (5.85 g), triethylamine (5 ml), and 4-dimethylaminopyridine (0.2 g) in dry N,N-dimethylformamide (15 ml) was stirred for 10 hours at ambient temperature.

The resulting mixture was poured into water (200 ml) and extracted with diethyl ether. The combined extracts were washed with water, dried, and evaporated under reduced pressure.

The oily residue was purified by column chromatography on silicia gel (100 g, elution by benzene and then chloroform) to give 4.90 g of methyl 2-O-methyl-5-O-trityl-α,β-D-xylofuranoside as an oil.

IR (film): 3500, 3050, 2900, 2800, 1595, 1490, 1445 $cm^{-1}$

(2) To a solution of the above compound (4.80 g) in dry N,N-dimethylformamide (24 ml) was added sodium hydride (0.82 g, 50% oil dispersion) in one portion. After stirring the mixture for one hour at ambient temperature, a solution of 1-bromooctadecane (4.18 g) in dry N,N-dimethylformamide (12 ml) was added dropwise thereto. The resulting mixture was stirred for 24 hours at ambient temperature and then poured into ice water (200 ml). The separated oil was extracted with diethyl ether.

The combined extracts were washed with water, dried, and evaporated under reduced pressure. The residue was purified by column chromatography on silica gel, (160 g, elution by benzene and then benzene/chloroform = $4^V/1^V$) to yield 3.3 g of methyl 2-O-methyl-3-O-octadecyl-5-O-trityl-β-D-xylofranoside as a colorless oil and 2.76 g of the α-isomer as a colorless oil.

β-isomer

IR (film): 3050, 3000, 1595, 1490, 1460, 1450 $cm^{-1}$

α-isomer

IR (film): 2930, 2850, 1460, 1445 $cm^{-1}$

(3) To a solution of methyl 2-O-methyl-3-O-octadecyl-5-O-trityl-β-D-xylofuranoside (3.2 g) in dry methylene chloride (32 ml) was added trifluoroacetic acid (3.2 ml) in one portion at ambient temperature. After stirring for 1.5 hours at the same temperature, the yellow solution was washed with water, aqueous sodium bicarbonate and water successively, dried, and evaporated under reduced pressure. The residue was triturated in n-hexane to remove triphenylcarbinol by filtration. The filtrate was evaporated and then the residue was purified by column chromatography on silica gel (40 g, elution by chloroform) to yield 1.21 g of methyl 2-O-methyl-3-O-octadecyl-β-D-xylofuranoside as a thick oil.

IR (film): 3500, 2910, 2850, 1465 $cm^{-1}$

(4) To a mixture of the above compound (1.15 g) and triethylamine (0.56 g) in dry methylene chloride (5 ml) was dropwise added a solution of 2-bromoethylphosphorodichloridate (1.33 g) in dry methylene chloride (2 ml) over a period of 10 minutes at 5°C. After the addition was complete, the mixture was stirred for 8 hours at ambient temperature and then cooled in an ice bath. To the cold solution was added dropwise a mixture of pyridine (3 ml) and water (1.5 ml) over a period of 2 minutes. The ice bath was removed and the mixture was stirred for one hour at ambient temperature to hydrolyze phosphorochloridate derivative and then evaporated to dryness. The residue was dissolved in a mixture of ethyl acetate (25 ml) and diethyl ether (25 ml). The solution was washed with 10% aqueous hydrochloric acid and brine, dried, and evaporated to yield 1.8 g of methyl 2-O-methyl-3-O-actadecyl-β-D-xylofuranoside 5-(2-bromoethyl phosphate) as a waxy solid.

IR (Nujol®): 1460, 1375, 1260 $cm^{-1}$

NMR ($CDCl_3$) ppm: 0.86 (m, 3H), 1.27 (s, 32H), 3.42 (s, 6H), 3.50 (m, 4H), 3.72 (m, 1H), 3.90 (dd, 1H, J = 8, 2.5Hz), 4.36 (m, 5H), 4.86 (s, 1H)

(5) A mixture of the above compound (1.7 g) and 30% aqueous trimethylamine (5.4 g) in methanol (11 ml) was stirred at 50°C for 7.5 hours. An insoluble material was filtered off and the filtrate was evaporated under reduced pressure. The residue was dissolved in 90% aqueous methanol (17 ml) was treated with silver acetate (1.13 g) for 1.5 hours with vigorous stirring. The precipitates were removed by filtration and washed with methanol. The combined filtrate and washings were evaporated to dryness. The resulting solid was chromatographed on silica gel (35 g, elution by $CHCl_3$:$CH_3OH$:$H_2O$ = 65:25:2) to give 1.0 g of methyl 2-O-methyl-3-O-octadecyl-β-D-xylofuranoside 5-(choline phosphate) which was purified by recrystallization from chloroform-acetone.

mp. 200°C (dec.)

IR (Nujol®): 3400, 1460, 1375, 1250 $cm^{-1}$

NMR ($CDCl_3$) ppm: 0.90 (m, 3H), 1.28 (s, 32H), 3.33 (s, 6H), 3.35 (m, 2H), 3.40 (s, 9H), 3.68 (m, 1H), 3.84 (m, 3H), 4.10 (m, 1H), 4.36 (m, 4H), 4.82 (s, 1H)

Anal. Calcd. for $C_{30}H_{62}NO_8P \cdot 2H_2O$: C; 57.03, H; 10.53, N; 2.22
Found: C; 56.15, H; 10.60, N; 2.18

Example 2

Methyl 2-O-methyl-3-O-octadecyl-β-D-xylofuranoside 5-[2-(1-pyridinio)ethyl phosphate] (0.75 g) was obtained by reacting methyl 2-O-methyl-3-O-octadecyl-β-D-xylofuranoside 5-(2-bromoethyl phosphate) (2.0 g) with pyridine (5 ml) in a similar manner to that of Example 1—(5).

mp. 110°C (dec.)

IR (Nujol®): 3360, 1640, 1495, 1250 $cm^{-1}$

NMR ($CD_3OD$) ppm: 0.90 (m, 3H), 1.30 (m, 32H), 3.37 (s, 3H), 4.00 (s, 3H), 3.1~4.1 (m, 6H) 4.2~4.5 (m, 3H), 4.78~5.00 (m, 3H), 8.00~9.1 (m, 5H)

Anal. Calcd. for $C_{32}H_{58}NO_8P \cdot H_2O$: C; 60.64, H; 9.54, N; 2.21
Found: C; 60.94, H; 9.31, N; 2.19

Example 3

Methyl 2-O-methyl-3-O-octadecyl-β-D-xylofuranoside 5-[2-(4-methyl-1-piperazinyl)ethyl phosphate] (1.69 g) was obtained as an oil by reacting methyl 2-O-methyl-3-O-octadecyl-β-D-xylofuranoside 5-(2-bromoethyl phosphate) (3.75 g) with 1-methylpiperazine (6.1 ml) in a similar manner to that of Example 1—(5).

IR (film): 3370, 2400, 1650, 1460, 1215 $cm^{-1}$

NMR ($CDCl_3$) ppm: 0.9 (m, 3H), 1.3 (m, 32H), 2.60 (s, 3H), 2.74~4.6 (m, 20H), 3.42 (s, 6H), 4.82 (s, 1H)

Anal. Calcd. for $C_{32}H_{65}N_2O_8P \cdot H_2O$ C; 58.69, H; 10.31, N; 4.28
Found: C; 58.99, H; 10.05 N; 4.34

Example 4

(1) Methyl 2-O-methyl-3-O-octadecyl-α-D-xylofuranoside (1.70 g) was obtained from methyl 2-O-methyl-3-O-octadecyl-5-O-trityl-α-D-xylofuranoside (3.89 g) in a similar manner to that of Example 1—(3).

mp. 65.5 to 66.5°C

IR (Nujol®): 3250, 3100, 1130 $cm^{-1}$

(2) Methyl 2-O-methyl-3-O-octadecyl-α-D-xylofuranoside 5-(2-bromoethyl phosphate) ( 2.07 g) was obtained as a waxy solid from the above compound (1.50 g) in a similar manner to that of Example 1—(4).

IR (Nujol®): 1120, 1070, 1010 $cm^{-1}$

(3) Methyl 2-O-methyl-3-O-octadecyl-α-D-xylofuranoside 5-(choline phosphate) (1.65 g) was obtained by reacting the above compound (2.04) g) with 30% aqueous trimethylamine (6.49 g) in a similar manner to that of Example 1—(5).

mp. 207°C (dec.)

IR (Nujol®): 3550, 3400, 3320, 1240, 1060 $cm^{-1}$

NMR ($CDCl_3$) ppm: 0.88 (m, 3H) 1.28 (m, 32H), 3.2~3.6 (m, 15H), 3.6~4.5 (m, 11H). 4.92 (d, 1H, J = 4Hz)

Anal. Calcd. for $C_{30}H_{62}NO_8P \cdot 2H_2O$: C; 57.03 H; 10,53, N; 2.22
Found: C; 57.60, H; 10.32, N; 2.22

Example 5

(1) 1,2-O-Isopropylidene-3-O-octadecyl-5-O-trityl-α-D-xylofuranose (22.55 g) was obtained as an oil from 1,2-O-isopropylidene-5-O-trityl-α-D-xylofuranose (21.6 g) in a similar manner to that of Example 1—(2).

IR (film): 3050, 3025, 2920, 2850, 1595, 1490, 1445, 1370 $cm^{-1}$

(2) 1,2-O-Isopropylidene-3-O-octadecyl-α-D-xylofuranose (12.7 g) was obtained as a waxy solid from the above compound (22.5 g) in a similar manner to that of Example 1—(3).

IR (Nujol®): 3500, 1460, 1375, 1080, 1005 $cm^{-1}$

(3) 1,2-O-Isopropylidene-3-O-octadecyl-α-D-xylofuranose 5-(2-bromoethyl phosphate) (6.36 g) was obtained as an oil from the above obtained compound (4.42 g) in a similar manner to that of Example 1—(4).

IR (film): 2910, 2850, 1460 $cm^{-1}$

(4) 1,2-O-Isopropylidene-3-O-octadecyl-α-D-xylofuranose 5-(choline phosphate) (3.37 g) was obtained from the above compound (6.25 g) in a similar manner to that of Example 1—(5).

mp. 217 to 218°C

IR (Nujol®): 3300, 1470, 1380, 1240, 1090, 1060, 1015 $cm^{-1}$

NMR ($CDCl_3$) ppm: 0.87 (m, 3H), 1.24 (s, 3H), 1.28 (s, 32H), 1.45 (s, 3H), 3.37 (s, 9H), 3.4~4.4 (m, 10H), 4.48 (d, 1H, J = 4Hz), 5.89 (d, 1H, J = 4Hz)

Anal. Calcd. for $C_{31}H_{62}NO_8P \cdot 2H_2O$: C; 57.83, H; 10.33, N; 2.18
Found: C; 57.21, H; 10.45, N; 2.13

Example 6

(1) 1,2:5,6-Di-O-cyclohexylidene-3-O-octadecyl-α-D-allofuranose (30.3 g) was obtained from 1,2:5,6-di-O-cyclohexylidene-α-D-allofuranose (23.8 g) in a similar manner to that of Example 1—(2).

mp. 43 to 44°C

IR (film): 2910, 2850, 1470, 1450, 1365 $cm^{-1}$

(2) A solution of the above compound (10.0 g) in a mixture of acetic acid (200 ml) and water (60 ml) was heated at 60°C for 4.5 hours. The solvents were evaporated below 40°C under reduced pressure. The residue was purified by column chromatography on silicagel (150 g, elution by benzene and then benzene: acetone = 10:1). The eluates containing the object compound were combined and concentrated to give 7.53 g of 1,2-O-cyclohexylidene-3-O-octadecyl-α-D-allofuranose as an oil.

IR (film): 3400, 2910, 2850, 1460, 1445 $cm^{-1}$

(3) To a stirred solution of the above compound (7.45 g) in tetrahydrofuran (300 ml) was added a solution of periodic acid (7.84 g) in water (45 ml) in one portion at ambient temperature.

The stirring was continued for 1.5 hours at the same temperature. The solvents were evaporated to dryness. The residue was extracted with diethyl ether.

The extracts were washed successively with water, an aqueous sodium bicarbonate solution and brine, dried and evaporated to give 7.25 g of crude product. The product was purified by column chromatography on silica gel (70 g, elution by benzene, benzene:chloroform = 1:1, and then chloroform) to yield 6.81 g of 1,2-O-cyclohexylidene-3-O-octadecyl-α-D-ribo-pentodialdo-1,4-furanose as an oil.

IR (film): 3450, 2920, 2850, 1735 $cm^{-1}$

(4) To a stirred solution of the above compound (5.5 g) in a mixture of methanol (27 ml) and tetrahydrofuran (27 ml) was added sodium borohydride (0.6 g) in one portion at ambient temperature. After the mixture was stirred for 10 minutes at the same temperature, the excess of sodium borohydride was decomposed by the addition of 10% aqueous hydrochloric acid. The solvents were evaporated under reduced pressure and the residue was extracted with chloroform. The extracts were combined and washed with water, an aqueous sodium bicarbonate solution, and brine successively, dried, and evaporated to dryness to yield 5.6 g of 1,2-O-cyclohexylidene-3-O-octadecyl-α-D-ribofuranose as a crystal.

mp. 35 to 37°C

IR (Nujol®): 3400, 3300, 1455, 1375 $cm^{-1}$

(5) 1,2-O-Cyclohexylidene-3-O-octadecyl-α-D-ribofuranose 5-(2-bromoethyl phosphate) (5.29 g) was obtained as an oil from the above compound (5.5 g) in a similar manner to that of Example 1—(4).

IR (film): 3450, 1460, 1455 $cm^{-1}$

(6) To a solution of the above compound (4.5 g) in a mixture of dry methanol (45 ml) and dry tetrahydrofuran (45 ml) was added conc. sulfuric acid (1.35 g) in one portion. The mixture was refluxed for 3 hours and then allowed to cool to ambient temperature. Barium carbonate (9.0 g) was added thereto. The

resultant suspension was stirred for one hour at ambient temperature and then filtered off. The filtrate was evaporated to give 5.0 g of crude product, which was purified by column chromotagraphy on silica gel (100 g, elution by chloroform and then 1~10% methanol in chloroform) to yield 3.85 g of methyl 3-O-octadecyl-β-D-ribofuranoside 5-(2-bromoethyl phosphate) as a waxy solid.

IR (Nujol®): 3420, 1470, 1225 $cm^{-1}$

(7) Methyl 3-O-octadecyl-β-D-ribofuranoside 5-(choline phosphate) (3.0 g) was obtained from the above compound (3.7 g) in a similar manner to that of Example 1—(5).

mp. 140°C

IR (Nujol®): 3400, 3200, 1460, 1245 $cm^{-1}$

NMR ($CDCl_3$) ppm: 0.86 (m, 3H), 1.28 (s, 32H), 3.2~4.5 (m, 23H), 4.88 (s, 1H).

| | | | |
|---|---|---|---|
| Anal. Calcd. for $C_{29}H_{60}NO_8P \cdot H_2O$: | C; 58.06, | H; 10.42, | N; 2.33 |
| Found: | C; 57.70, | H; 10.86, | N; 2.33 |

Example 7

(1) To a stirred mixture of 1,2-O-isopropylidene-3-amino-3-deoxy-α-D-ribofuranose (3.8 g) and triethylamine (4 ml) in dry benzene (38 ml) was dropwise added, a solution of octadecanoyl chloride (6.08 g) in dry benzene (10 ml) over a period of one hour in an ice bath. After stirring for 3 hours at ambient temperature, the mixture was washed successively with water, an aqueous sodium hydroxide solution, and water, dried, and evaporated to give crude product (8.28 g), which was purified by column chromatography on silica gel (160 g, elution by chloroform and then 1% methanol in chloroform) to yield 3.29 g of 1,2-O-isopropylidene-3-octadecanoylamino-3-deoxy-α-D-ribofuranose as a crystal.

mp. 74 to 76°C

IR (Nujol®): 3350, 1650, 1530, 1460, 1380, 1255, 1205 $cm^{-1}$

(2) 1,2-O-Isopropylidene-3-octadecanoylamino-3-deoxy-α-D-ribofuranose 5-(2-bromoethyl phosphate) (3.21 g) was obtained as a waxy solid from the above compound (3.2 g) in a similar manner to that of Example 1—(4).

IR (Nujol®): 3300, 1645, 1540, 1460, 1380, 1255 $cm^{-1}$

(3) 1,2-O-Isopropylidene-3-octadecanoylamino-3-deoxy-α-D-ribofuranose 5-(choline phosphate) (1.90 g) was obtained from the above compound (2.6 g) in a similar manner to that of Example 1—(5).

mp. 211 to 218°C (dec.)

IR (Nujol)®: 3300, 1645, 1560, 1460, 1375, 1245 $cm^{-1}$

NMR ($CDCl_3$) ppm: 0.86 (m, 3H), 1.27 (m, 33H), 1.51 (s, 3H), 2.20 (m, 2H), 3.36 (s, 9H), 3.6~4.4 (m, 8H), 4.60 (m, 1H), 5.82 (d, 1H, J = 4Hz), 6.44 (m, 1H)

| | | | |
|---|---|---|---|
| Anal. Calcd. for $C_{31}H_{61}N_2O_8P \cdot 3/2H_2O$: | C; 57.47, | H; 9.96, | N; 4.32 |
| Found: | C; 57.82, | H; 10.21, | N; 4.34 |

Example 8

(1) Methyl 3,5-di-O-benzyl-2-O-octadecyl-β-D-xylofuranoside (6.1 g) was obtained as an oil from methyl, 3,5-di-O-benzyl-β-D-xylofuranoside (6.0 g) in a similar manner to that of Example 1—(2).

IR (film): 3010, 2900, 2850, 1450, 1110, 1060 $cm^{-1}$

(2) A solution of the above obtained compound (6.0 g) in a mixture of methanol (120 ml), tetrahydrofuran (60 ml), and acetic acid (36 ml) containing palladium on charcoal (10%, 3.0 g) was hydrogenated at 4.05 bar (4 atm) hydrogen pressure for 24 hours. The catalyst was removed by filtration followed by washing with chloroform. The combined filtrate and washings were evaporated under reduced pressure to yield 4.5 g of crude methyl 2-O-octadecyl-α,β-D-xylofuranoside as a waxy solid, which was used in the following step without further purification.

IR (Nujol®): 3370, 1465, 1375, 1110, 1045 $cm^{-1}$

(3) Methyl 2-O-octadecyl-5-O-trityl-β-D-xylofuranoside (3.95 g) was obtained as an oil from the above compound (4.44 g) in a similar manner to that of Example 1—(1).

IR (film): 3500, 3050, 3010, 2910, 2840, 1595, 1490, 1465, 1445 $cm^{-1}$

(4) A mixture of the above compound (3.9 g), acetic anhydride (2 ml) and dry pyridine (4 ml) was heated at 80°C for 2 hours. The resultant mixture was poured into water (20 ml) and the separated oil was extracted with diethyl ether. The extract was washed with 5% aqueous hydrochloric acid, water, aqueous sodium bicarbonate, water, and brine successively. After drying, the solvent was evaporated to give 4.1 g of methyl 3-O-acetyl-2-O-octadecyl-5-O-trityl-β-D-xylofuranoside as an oil.

IR (film): 3050, 3010, 2900, 2830, 1740, 1235 $cm^{-1}$

(5) Methyl 3-O-acetyl-2-O-octadecyl-β-D-xylofuranoside (1.67 g) was obtained as a waxy solid from the above obtained compound (4.0 g) in a similar manner to that of Example 1—(3).

IR (Nujol®): 3430, 1740, 1710, 1460, 1375, 1110, 1065 $cm^{-1}$

(6) Methyl 3-O-acetyl-2-O-octadecyl-β-D-xylofuranoside 5-(2-bromoethyl phosphate) (2.22 g) was obtained as an oil from the above compound (1.6 g) in a similar manner to that of Example 1—(4).

IR (film): 2900, 2850, 1740 $cm^{-1}$

(7) A mixture of the above compound (2.15 g) and 30% aqueous trimethylamine (6.6 g) in methanol (13 ml) was stirred at 50°C for 9 hours. An insoluble material was filtered off and the filtrate was evaporated

under reduced pressure. The residue was dissolved in methanol (50 ml). To the solution was added 28% methanolic sodium methoxide (2 ml) and the mixture was stirred for 6 hours at ambient temperature. The mixture was evaporated and the residue was dissolved in a mixture of methanol (10 ml), chloroform (5 ml), and water (5 ml). The solution was adjusted to pH 2 with 10% hydrochloric acid and passed through a mixed ion-exchange column, containing Amberlite® IRC-50 ($H^+$) (40 ml) and IR-45 ($OH^-$) (40 ml). The eluates were evaporated to dryness to give 2.1 g of crude product, which was further purified by column chromatography on silica gel (42 g) (elution by $CHCl_3$:$CH_3OH$:$H_2O$ = 65:25:4) and recrystallization from chloroform-acetone to give 0.33 g of methyl 2-O-octadecyl-β-D-xylofuranoside 5-(choline phosphate).

mp. 217 to 220°C.

IR (Nujol®): 3200, 1460, 1200, 1045 $cm^{-1}$

NMR ($CDCl_3$) ppm: 0.87 (m, 3H), 1.25 (s, 32H), 3.23 (s, 9H), 3.35 (s, 3H) 3.2~4.6 (m, 11H), 4.73 (s, 1H)

Anal. Calcd. for $C_{29}H_{60}NO_8P \cdot 5/2H_2O$: C; 55.57, H; 10.45, N; 2.23

Found: C; 55.43, ; H, 10.33, N; 2.07

Example 9

(1) To a solution of 5-O-benzyl-1,2-O-isopropylidene-3-O-methyl-α-D-xylofuranose (7.0 g) in methanol (21 ml) was added conc. hydrochloric acid (0.2 ml). The mixture was refluxed for 2 hours and then allowed to cool to ambient temperature. To the resultant mixture was added barium carbonate (2.0 g). The suspension was stirred for 2 hours and filtered. The filtrate was evaporated under reduced pressure. The oily residue was purified by column chromatography on silica gel (150 g, elution by $CHCl_3$, 1~3% $CHCl_3$ in methanol) to give 2.4 g of methyl 5-O-benzyl-3-O-methyl-α-D-xylofuranoside and 3.25 g of methyl 5-O-benzyl-3-O-methyl-β-D-xylofuranoside.

α-isomer, oil

NMR ($CCl_4$) ppm: 2.44 (d, 1H, J = 7Hz), 3.36 (s, 3H), 3.44 (s, 3H), 3.58 (m, 3H), 3.98 (m, 1H), 4.18 (m, 1H), 4.45 (s, 2H), 4.79 (d, 1H, J = 5Hz), 7.21 (m, 5H)

β-isomer, oil

NMR ($CCl_4$) ppm: 2.48 (brs, 1H) 3.26 (s, 3H), 3.30 (s, 3H), 3.52 (m, 3H), 3.98 (m, 1H), 3.94 (m, 1H), 4.48 (s, 2H), 4.63 (s, 1H), 7.21 (m, 5H)

(2) Methyl-5-O-benzyl-3-O-methyl-2-O-octadecyl-α,β-D-xylofuranoside (9.5 g) was obtained as an oil from the mixture of above α and β isomer (5.9 g) in a similar manner to that of Example 1—(2).

IR (film): 2900, 1820, 1460, 1360, 1110 $cm^{-1}$

(3) Methyl 3-O-methyl-2-O-octadecyl-α,β-D-xylofuranoside (3.92 g) was obtained from the above compound (9.4 g) in a similar manner to that of Example 8—(2).

IR (film): 3350, 1460, 1375, 1120, 1065 $cm^{-1}$

(4) Methyl 3-O-methyl-2-O-octadecyl-α,β-D-xylofuranoside 5-(2-bromoethyl phosphate) (5.15 g) was obtained as an oil from the above compound (3.80 g) in a similar manner to that of Example 1—(4).

IR (film): 2900, 2840, 1460 $cm^{-1}$

(5) Methyl 3-O-methyl-2-O-octadecyl-α,β-D-xylofuranoside 5-(choline phosphate) (3.42 g) was obtained from the above compound (5.0 g) in a similar manner to that of Example 1—(5).

mp. 220°C

IR (Nujol®): 3350, 1645, 1460, 1375 $cm^{-1}$

NMR ($CDCl_3$) ppm: 0.87 (m, 3H), 1.28 (s, 32H), 3.3~4.5 (m, 26H), 4.80~4.92 (m, 1H)

Anal. Calcd. for $C_{30}H_{62}NO_8P \cdot 2H_2O$: C; 57.03, H; 10.53, N; 2.22

Found: C; 57.00, ; 10.60, N; 2.16

**Claims**

1. A compound of the formula:

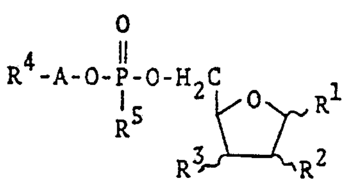

[I]

wherein $R^1$, $R^2$, and $R^3$ are each hydroxy, $C_1$—$C_{25}$ alkoxy, $C_1$—$C_{25}$ alkanoylamino or hydroxy group which is protected by a conventional protective group for hydroxy;

A is $C_2$—$C_6$alkylene;

$R^4$ is tri($C_1$—$C_6$alkyl)-ammonio, 5,6 or 7-membered cyclic ammonio or 5,6 or 7-membered cyclic amino; and

$R^5$ is oxido anion or hydroxy;

and pharmaceutically acceptable salts thereof.

2. A compound of claim 1, wherein $R^1$, $R^2$ and $R^3$ are each $C_1$—$C_{25}$ alkoxy.

3. A compound of claim 2, wherein $R^4$ is tri($C_1$—$C_6$alkyl)ammonio.

4. The compound of claim 3, which is methyl 2-O-methyl-3-O-octadecyl-β-D-xylofuranoside 5-(choline phosphate).

5. The compound of claim 3, which is methyl 2-O-methyl-3-O-octadecyl-α-D-xylofuranoside 5-(choline phosphate).

6. The compound of claim 3, which is methyl 3-O-methyl-2-O-octadecyl-α,β-D-xylofuranoside 5-(choline phosphate).

7. A compound of claim 2, wherein $R^4$ is 5,6 or 7-membered cyclic ammonio.

8. The compound of claim 7, which is methyl-2-O-methyl-3-O-octadecyl-β-D-xylofuranoside 5-[2-(1-pyridinio)ethyl phosphate].

9. A compound of claim 2, wherein $R^4$ is 5,6 or 7-membered cyclic amino.

10. The compound of claim 9, which is methyl 2-O-methyl-3-O-octadecyl-β-D-xylofuranoside 5-[2-(4-methyl-1-piperazinyl)ethyl phosphate].

11. A compound of claim 1, wherein $R^1$ and $R^3$ are each $C_1$—$C_{25}$alkoxy, $R^2$ is hydroxy and $R^4$ is tri($C_1$—$C_6$alkyl)ammonio.

12. The compound of claim 11, which is methyl 3-O-octadecyl-β-D-ribofuranoside 5-(choline phosphate).

13. A compound of claim 1, wherein $R^1$ and $R^2$ are each $C_1$—$C_{25}$alkoxy, $R^3$ is hydroxy and $R^4$ is tri($C_1$—$C_6$alkyl)ammonio.

14. The compound of claim 13, which is methyl 2-O-octadecyl-β-D-xylofuranoside 5-(choline phosphate).

15. A compound of claim 1, wherein $R^1$ and $R^2$ are each hydroxy group which is protected by a conventional protective group for hydroxy, and $R^4$ is tri($C_1$—$C_6$alkyl)ammonio.

16. A compound of claim 15, wherein $R^3$ is $C_1$—$C_{25}$alkoxy.

17. A compound of claim 15, wherein $R^3$ is $C_1$—$C_{25}$alkanoylamino.

18. A process for preparing a compound of the formula:

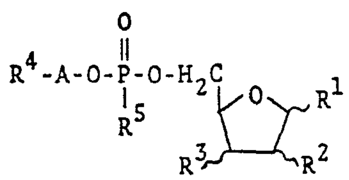

[I]

wherein $R^1$, $R^2$, $R^3$, A, $R^4$ and $R^5$ are each as defined in claim 1 or pharmaceutical salts thereof, which comprises by reacting a compound of the formula:

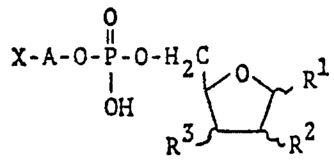

[II]

wherein $R^1$, $R^2$, $R^3$, A and $R^4$ are each as defined above; and

X is halogen;

or its salt with a tri($C_1$—$C_6$alkyl)amine or 5,6 or 7-membered cyclic amine or a salt thereof.

19. A pharmaceutical composition comprising an effective amount of a compound as claimed in claim 1 and a pharmaceutically acceptable, substantially nontoxic carrier or excipient.

**Patentansprüche**

1. Verbindung der Formel:

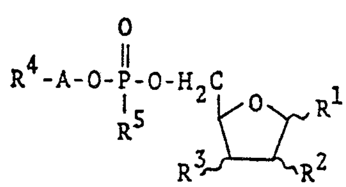

[I]

worin bedeuten:

$R^1$, $R^2$ und $R^3$ jeweils eine Hydroxy-, $C_1$—$C_{25}$-Alkoxy-, $C_1$—$C_{25}$-Alkanoylamino- oder Hydroxygruppe, die durch eine konventionelle Schutzgruppe für Hydroxy geschützt, ist;

A $C_2$—$C_6$-Alkylen;

$R^4$ Tri-($C_1$—$C_6$-alkyl)-ammonio, 5,6 oder 7-gliedriges cyclisches Ammonio oder 5,6 oder 7-gliedriges cyclisches Amino; und

$R^5$ ein Oxidonanion oder Hydroxy; sowie ihre pharmazeutisch akzeptablen Salze.

2. Verbindung nach Anspruch 1, worin $R^1$, $R^2$ und $R^3$ jeweils $C_1$—$C_{25}$-Alkoxy bedeuten.

3. Verbindung nach Anspruch 2, worin $R^4$ Tri-($C_1$—$C_4$-alkyl)ammonio bedeutet.

4. Verbindung nach Anspruch 3, bei der es sich handelt um Methyl-2-O-methyl-3-O-octadecyl-β-D-xylofuranosid-5-(cholin-phosphat).

5. Verbindung nach Anspruch 3, bei der es sich handelt um Methyl-2-O-methyl-3-O-octadecyl-α-D-xylofuranosid-5-(cholin-phosphat).

6. Verbindung nach Anspruch 3, bei der es sich handelt um Methyl-3-O-methyl--2-O-octadecyl-α,β-D-xylofuranosid-5-(cholin-phosphat).

7. Verbindung nach Anspruch 2, worin $R^4$ 5,6 oder 7-gliedriges cyclisches Ammonio bedeutet.

8. Verbindung nach Anspruch 7, bei der es sich handelt um Methyl-2-O-methyl-3-O-octadecyl-β-D-xylofuranosid-5-[2-(1-pyridinio)ethyl-phosphat].

9. Verbindung nach Anspruch 2, worin $R^4$ 5,6 oder 7-gliedriges cyclisches Amino bedeutet.

10. Verbindung nach Anspruch 9, bei der es sich handelt um Methyl-2-O-methyl-3-O-octadecyl-β-D-xylofuranosid-5-[2-(4-methyl-1-piperazinyl)ethyl-phosphat].

11. Verbindung nach Anspruch 1, worin $R^1$ und $R^3$ jeweils $C_1$—$C_{25}$-Alkoxy, $R^2$ Hydroxy und $R^4$ Tri-($C_1$—$C_6$-alkyl)ammonio bedeuten.

12. Verbindung nach Anspruch 11, bei der es sich handelt um Methyl-3-O-octadecyl-β-D-ribofuranosid-5-(cholin-phosphat).

13. Verbindung nach Anspruch 1, worin $R^1$ und $R^2$ jeweils $C_1$—$C_{25}$-Alkoxy, $R^3$ Hydroxy und $R^4$ Tri-($C_1$—$C_6$-alkyl)ammonio bedeuten.

14. Verbindung nach Anspruch 13, bei der es sich handelt um Methyl-2-O-octadecyl-β-D-xylofuranosid-5-(cholin-phosphat).

15. Verbindung nach Anspruch 1, worin $R^1$ und $R^2$ jeweils eine Hydroxygruppe bedeuten, die durch eine konventionelle Schutzgruppe für Hydroxy geschützt ist, und worin $R^4$ Tri-($C_1$—$C_6$-alkyl)ammonio bedeutet.

16. Verbindung nach Anspruch 15, worin $R^3$ $C_1$—$C_{25}$-Alkoxy bedeutet.

17. Verbindung nach Anspruch 15, worin $R^3$ $C_1$—$C_{25}$-Alkanoylamino bedeutet.

18. Verfahren zur Herstellung einer Verbindung der Formel

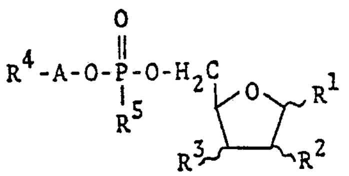

[I]

worin $R^1$, $R^2$, $R^3$, A, $R^4$ und $R^5$ jeweils wie in Anspruch 1 definiert sind, oder ihrer pharmazeutisch akzeptablen Salze, das unfaßt die Umsetzung einer Verbindung der Formel

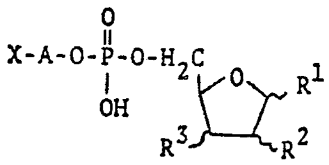

[II]

worin $R^1$, $R^2$, $R^3$, A und $R^4$ jeweils wie oben definiert sind und X Halogen bedeutet, oder ihres Salzes mit einem Tri-($C_1$—$C_6$-alkyl)amin oder 5,6 oder 7-gliedrigen cyclischen Amin oder einem Salz davon.

19. Pharmazeutische Zusammensetzung, die enthält eine wirksame Menge einer Verbindung nach Anspruch 1 und einen pharmazeutisch akzeptablen, im wesentlichen nicht-toxischen Träger oder Hilfsstoff.

**Revendications**

1. Composé de formule:

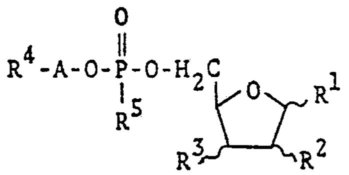

[I]

dans laquelle

$R^1$, $R^2$ et $R^3$ représentent chacum un groupe hydroxy, alcoxy en $C_1$ à $C_{25}$, alcanoylamino en $C_1$ à $C_{25}$ ou un groupe hydroxy qui est protégé par un groupe classique de protection des groupes hydroxy;

A représente un groupe alkylène en $C_2$ à $C_6$;

$R^4$ représente un groupe tri(alkyl en $C_1$ à $C_6$)ammonio, un groupe ammonio cyclique comportant 5, 6 ou 7 chaînons, ou un groupe amino cyclique comportant 5, 6 ou 7 chaînons, et $R^5$ est un anion oxydo ou hydroxy; et ses sels pharmaceutiquement acceptables.

2. Composé selon la revendication 1, dans lequel $R^1$, $R^2$ et $R^3$ représentent chacun groupe alcoxy en $C_1$ à $C_{25}$.

3. Composé selon la revendication 2, dans lequel $R^4$ représente un groupe tri(alkyl en $C_1$ à $C_6$)ammonio.

4. Composé selon la revendication 3, qui est le 5-(choline phosphate) de méthyle et de 2-O-méthyl-3-O-octadécyl-β-D-xylofurannoside.

5. Composé selon la revendication 3, qui est le 5-(choline phosphate) de méthyle et de 2-O-méthyl-3-O-octadécyl-α,-D-xylofurannoside.

6. Composé selon la revendication 3, qui est le 5-(choline phosphate) de méthyle et de 3-O-méthyl-2-O-Octadécyl-α,β-D-xylofurannoside.

7. Composé selon la revendication 2, dans lequel $R^4$ représente un groupe ammonio cyclique comportant 5, 6 ou 7 chaînons.

8. Composé selon la revendication 7, qui est le 5-[2-(1-pyridinio)éthyl phosphate] de méthyle et de 2-O-méthyl-3-O-octadécyl-β-D-xylofurannoside.

9. Composé selon la revendication 2, dans lequel $R^4$ représente un groupe amino cyclique comportant 5, 6 ou 7 chaînons.

10. Composé selon la revendication 9, qui est le 5-[2-(4-méthyl-1-pipérazinyl)éthyl phosphate] de méthyle et le 2-O-méthyl-3-O-octadécyl-β-D-xylofurannoside.

11. Composé selon la revendication 1, dans lequel $R^1$ et $R^3$ représentent chacum un groupe alcoxy $C_1$ à $C_{25}$, $R^2$ représente un groupe hydroxyle et $R^3$ représente un groupe tri(alkyl en $C_1$ à $C_6$)ammonio.

12. Composé selon la revendication 11, qui est le 5-(choline phosphate) de méthyle et de 3-O-octadécyl-β-D-ribofurannoside.

13. Composé selon la revendication 1, dans lequel $R^1$ et $R^2$ représentent chacun un groupe alcoxy en $C_1$ à $C_{25}$, $R^3$ est un groupe hydroxy et $R^4$ est un groupe tri(alkyl en $C_1$ à $C_6$)ammonio.

14. Composé selon la revendication 13, qui est le 5-(choline phosphate) de méthyle et de 2-O-octadécyl-β-D-xylofurannoside.

15. Composé selon la revendication 1, dans lequel $R^1$ et $R^2$ représentent chacum un groupe hydroxy qui est protégé par un groupe classique de protection de groupe hydroxy, et $R^4$ est un groupe tri(alkyl en $C_1$ à $C_6$)ammonio.

16. Composé selon la revendication 15, dans lequel $R^3$ représente un groupe alcoxy en $C_1$ à $C_{25}$.

17. Composé selon la revendication 15, dans lequel $R^3$ représente un groupe alcanoylamino en $C_1$ à $C_{25}$.

18. Procédé pour préparer un composé de formule:

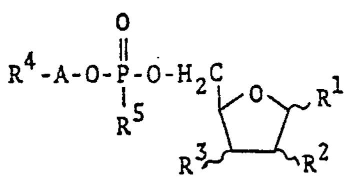

[I]

dans laquelle $R^1$, $R^2$, $R^3$, A, $R^4$ et $R^5$ sont chacun tels que définis à la revendication 1, ou ses sels pharmaceutiques, qui comprend la réaction d'un composé de formule:

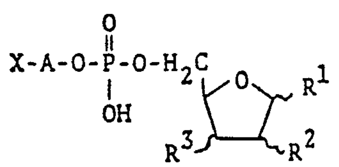

[II]

dans laquelle $R^1$, $R^2$, $R^3$, A, et $R^4$ sont chacun comme définis ci-dessus et X est un atome d'halogène; ou d'un de ses sels avec une trialkyl (en $C_1$ à $C_6$) amine ou une amine cyclique comportant 5, 6 ou 7 chaînons, ou un de leurs sels.

19. Composition pharmaceutique comprenant une quantité efficace d'un composé tel que revendiqué à la revendication 1 et un support, véhicule ou excipient pharmaceutiquement acceptable et essentiellement non toxique.